# EUROPEAN PATENT SPECIFICATION

(11) **EP 3 085 397 B1**
(45) Date of publication and mention of the grant of the patent: **19.06.2019**
(21) Application number: 16166609.4
(22) Date of filing: 22.04.2016
(51) Int. Cl.: A61L 31/04, A61L 24/04, C08L 67/04, C08L 71/02

(54) **ADHESIVE COMPOSITIONS**
KLEBSTOFFZUSAMMENSETZUNGEN
COMPOSITIONS ADHÉSIVES

(30) Priority: 23.04.2015 US 201562151455 P; 17.03.2016 US 201615072402
(43) Date of publication of application: 26.10.2016
(73) Proprietor: Covidien LP, Mansfield, MA 02048 (US)
(72) Inventor: BENNETT, Steven L., Cheshire, CT 06410 (US); SKALLA, Walter, Old Lyme, CT 06371 (US); VERNLUND, Lauren, Berlin, CT 06037 (US); MAST, Nathaniel, Hamden, CT 06517 (US)
(74) Representative: Soames, Candida Jane

(56) References cited:
- US-A1- 2003 108 511
- US-A1- 2006 210 602

## Description

### BACKGROUND

The present disclosure is related to biocompatible adhesives, which may be used by themselves for adhering/sealing tissue, or as coatings for medical devices. Coatings have been applied to medical devices to impart lubricious, non-thrombogenic, adhesive, and/or anti-adhesive properties and serve as depots for bioactive agent release. Adherence of these coatings to the substrate used to form the medical device may prove difficult, with delamination occurring in some cases. In addition, depending upon the materials used to form the coatings and/or the intended purpose of the coating, issues may arise with adherence of the coating to not only the device, but also to the tissue to which the device is to be applied.

US 2006/210602 discloses an adhesive composition comprising a branched polyethylene glycol such as a tetrafunctionally activated PEG succinimidyl glutarate in combination with a linear polyethylene glycol like a multi-amino PEG. The composition might further include other components such as for instance tensile strength enhancers like polyglycolide:polylactide-containing fibers.

Suitable compositions for tissue adhesives, including compositions suitable for adherence of medical devices to tissue, as well as methods for their manufacture and use, remain desirable.

### SUMMARY

The present disclosure provides biocompatible adhesive compositions which may be used by themselves or, in embodiments, as coatings on medical devices to assist the adherence of the medical device to tissue.

In embodiments, a biocompatible adhesive of the present disclosure includes a first component including at least one linear polyethylene glycol having a molecular weight from 1,000 to 10,000 Daltons, in combination with at least one multi-arm polyethylene glycol having a molecular weight from 1,000 to 3,000 Daltons; and a second component including a lactone. In embodiments, the first component includes the at least one linear polyethylene glycol and the at least one multi-arm polyethylene glycol at a ratio from 99:1 to 80:20. In other embodiments, the first component includes the at least one linear polyethylene glycol and the at least one multi-arm polyethylene glycol at a ratio from 98:2 to 88:12. In embodiments, the multi-arm polyethylene glycol has from four to twelve arms. In embodiments, the ratio of the ether to the lactone is about 2:1.

As noted above, compositions of the present disclosure may be used by themselves as a tissue adhesive/sealant, or may be used as a coating on a medical device to enhance adherence of the medical device to tissue.

For example, in embodiments, compositions of the present disclosure may be used as coatings on medical devices such as hernia patches. A hernia patch of the present disclosure may include, in embodiments a mesh substrate having a surface; and a coating on at least a portion of the surface of the mesh substrate, the coating including a first component including at least one linear polyethylene glycol having a molecular weight from 1,000 to 10,000 Daltons, in combination with at least one multi-arm polyethylene glycol having a molecular weight from 1,000 to 3,000 Daltons, and a second component including a lactone. In embodiments, the first component includes the at least one linear polyethylene glycol and the at least one multi-arm polyethylene glycol at a ratio from 99:1 to 80:20. In other embodiments, the first component includes the at least one linear polyethylene glycol and the at least one multi-arm polyethylene glycol at a ratio from 98:2 to 88:12. In embodiments,_the multi-arm polyethylene glycol has from four to twelve arms. In embodiments, the ratio of the ether to the lactone is about 2:1.

Methods of the present disclosure include application of the compositions of the present disclosure to tissue. In embodiments, methods include contacting a medical device, such as a hernia patch including a composition of the present disclosure, to tissue, re-positioning the hernia patch, as necessary, to an optimal location on the tissue; and permitting the coating to adhere the hernia patch to tissue. Re-positioning the hernia patch includes, in embodiments, contacting the coating to tissue, temporarily affixing the patch to the tissue, removing the patch from the tissue, and again contacting the coating to the tissue, thereby placing the patch in the optimal location.

### BRIEF DESCRIPTION OF THE DRAWINGS

The accompanying drawings, which are incorporated in and constitute a part of this specification, illustrate embodiments of the disclosure and, together with a general description of the disclosure given above, and the exemplary embodiments described below, serve to explain the principles of the present disclosure.
FIG. 1 is an illustration of a hernia implant formed of a mesh including a composition of the present disclosure as a coating; and
FIG. 2 is an illustration of an alternate hernia implant formed of a mesh including a composition of the present disclosure as a coating.

### DETAILED DESCRIPTION

The present disclosure provides coatings suitable for use with medical devices. To be effective, the compositions of the present disclosure are made from materials that meet biological and physical requirements. The materials should be, at least in part, bioresorbable, nontoxic, noncarcinogenic, nonantigenic, and should demonstrate favorable mechanical properties such as flexibility, suturability in some cases, and the ability to be re-positioned on tissue to allow for optimal placement of a medical device thereto. For certain indications, for example hernia repair, the coating should be strong enough to provide at least temporary fixation of a hernia mesh to tissue and, in some cases, removal from the tissue and re-adherence to the tissue as the hernia mesh is repositioned to an optimal location.

In embodiments, compositions of the present disclosure are formed of multiple polymers, and have adhesive characteristics, sometimes referred to herein as tackiness, that allow them to be attached to tissue, and repositioned as needed and/or desired. Compositions of the present disclosure may be used by themselves as tissue adhesives and/or sealants, or may be applied to medical devices as coatings to enhance adherence of the medical device to tissue. Where used as a coating, the coatings of the present disclosure permit a medical device to which they are applied to be placed in an optimal location in vivo prior to its fixation. Compositions of the present disclosure may be used as, or applied to other devices used as, hemostatic aids, tissue repair/reinforcement devices, including meshes and films for hernia repair, slow-release drug delivery systems, and the like.

Coatings of the present disclosure may be used to temporarily fix devices to tissue, allowing proper positioning of the device prior to final attachment of the device to tissue. In embodiments, devices possessing the coatings of the present disclosure may be affixed to tissue by application of an additional fixation device, such as staples, sutures, clips, etc. In other embodiments, coatings of the present disclosure may be utilized to fix devices to tissue without the need for any additional fixation device, i.e., they may fix the device to tissue on their own.

The compositions of the present disclosure include at least two polymeric components. The first component is formed from at least one ether, in some cases a polyether. Suitable ethers and/or polyethers which may be used as the first component include, but are not limited to, alkylene oxides, including ethylene oxide and propylene oxide; alkyl substituted ethylene oxides such as ethyl, propyl, and butyl substituted ethylene oxide; polyalkylene oxides such as polyethylene oxide ("PEO"), polypropylene oxide ("PPO"), polyethylene oxide-co-polypropylene oxide ("PEO-PPO"), co-polyethylene oxide block or random copolymers; alkylene glycols including ethylene glycol and polyethylene glycol ("PEG"); polytetramethylene ether glycol, combinations thereof, and the like.

In embodiments a PEG may be used as the first component. The PEG may be linear, or it may have a star configuration, i.e., multiple arms emanating from a core. The number of arms may vary, in embodiments from about four arms to about twelve arms, in embodiments from about six arms to about eight arms.

As noted above, the compositions of the present disclosure also include at least a second component. The second component may include one or more recurring lactone monomers and/or polymers.

Suitable lactones which may be utilized in forming the second component include, but are not limited to, lactide, glycolide, caprolactones, valerolactones, dioxanones, dioxepanones, trimethylene carbonates, propiolactones, butyrolactone, combinations thereof, and the like.

In embodiments, the second component may be made of a quaternary polymer of glycolide, trimethylene carbonate, caprolactone, and L-lactide, wherein the polymer includes from 62% to 72% by weight glycolide, in embodiments from 67% to 71% by weight glycolide, from 1% to 10% by weight trimethylene carbonate, in embodiments from 6% to 8% by weight trimethylene carbonate, from 12% to 20% by weight caprolactone, in embodiments from 15% to 18% by weight caprolactone, and from 1% to 10% by weight L-lactide, in embodiments from 6% to 8% by weight L-lactide.

In embodiments, the second component may be a synthetic polyester composed of glycolide, caprolactone, trimethylene carbonate and lactide. The polyester may be a random copolymer possessing 17% caprolactone, 7% lactide, 7% trimethylene carbonate, and 69% glycolide.

In other embodiments, the second component may be made of a glycolide-L-lactide copolymer wherein the copolymer includes from about 87% to about 99% by weight glycolide, in embodiments from about 89% to about 93% by weight glycolide, and from about 4% to about 13% by weight of L-lactide, in embodiments from about 7% to about 11% by weight of L-lactide.

In other embodiments, the second component may be made of 100% glycolide or 100% polydioxanone.

The compositions of the present disclosure may be prepared by transesterification of the first component, preferably present in an excess amount, with the second component. In embodiments, the first component and second component are thus linked by ester bonds. The controlled synthesis of high molecular weight polyesters requires the complete absence, or at least severe limitation, of side inter- and intramolecular transesterification reactions, which perturb chain propagation and broaden the molecular weight distribution. Transesterification is the random scission and recombination of molecules induced by heating, where recombination does not necessarily take place between the same fragments which together made up the initial molecule. There are two types of transesterification: intermolecular and intramolecular. Intramolecular transesterification, or "back-biting", results in polymer degradation and the formation of cyclic oligomers. Intermolecular transesterification affects the sequences of different polymeric segments and prevents the formation of a block structure in the polymer. Both types of transesterification broaden the molecular weight distribution.

Suitable weight average molecular weights (Mw) of the first components is from 1,000 to 10,000 Daltons. The first component is a polyethylene glycol ("PEG"), suitable PEGs include those commercially available from a variety of sources under the designations PEG 200, PEG 400, PEG 600, PEG 900, PEG 1,000, PEG 1,500, PEG 2,000, PEG 3,350, PEG 4,600, PEG 5,000 and PEG 10,000.

In embodiments, combinations of ethers, in embodiments PEG, may be used as the first component. For example, in embodiments, a linear PEG with a weight average molecular weight of about 4,600 (PEG 4,600) may be combined with a multi-arm PEG, in embodiments having 4 arms, having a weight average molecular weight of from about 1,000 (PEG 1,000) to about 2,000 (PEG 2,000).

Where combinations of ethers such as PEG are utilized, the ratio of first PEG to second PEG is from 99:1 to 80:20, encompassing any and all ratios therebetween, in embodiments from 98:2 to 88:12, in embodiments from about 96:4 to about 90:10, in embodiments about 90:10. In embodiments, the ratio of first PEG to second PEG may be from about 99:1 to about 90:10.

Suitable weight average molecular weights (Mw) of the second components may be from about 1,000 to about 150,000 Daltons, in embodiments from about 5,000 to about 100,000 Daltons, in embodiments from about 10,000 to about 50,000 Daltons.

In some cases, the first component and the second component may be produced by a reaction in the presence of a catalyst, such as metal chlorides, including stannous chloride (SnCl₄); metal alkoxides, including aluminum alkoxides which may be prepared by a reaction of triethylaluminum with an alcohol; zinc alkoxides, including zinc acetate; tin alkoxides, including, stannous octoate (tin(ii) bis-(2-ethylhexanoate)); and combinations thereof.

The amount of catalyst will vary depending upon the amount of first component and second component, but may be from about 0.01 to about 0.1% by weight of the reactants (the first component and second component), in embodiments, from about 0.02 to about 0.09% by weight of the reactants.

Synthesis may occur at an elevated temperature, in embodiments from about 120°C to about 220°C, in other embodiments from about 140°C to about 170°C.

In embodiments, the first component may be a multi-arm PEG, such as a 4 arm PEG having a weight average molecular weight of about 10000. The PEG is combined with a copolymer including 17% caprolactone, 7% lactide, 7% trimethylene carbonate, and 69% glycolide, and about 0.04% by weight of stannous octoate, at a temperature of about 190°C, resulting in a homogeneous material having a glass transition temperature (Tg) of about -33 to -39°C, and a melting temperature (Tm) of about 38-42°C.

In other embodiments, the first component may be a multi-arm PEG, such as a 4 arm PEG having a weight average molecular weight of about 2000. The PEG is combined with a copolymer including 17% caprolactone, 7% lactide, 7% trimethylene carbonate, and 69% glycolide, and about 0.04% by weight of stannous octoate, at a temperature of about 190°C.

The types and amounts of the first and second components making up the compositions of the present disclosure may vary according to the intended use of the compositions so formed. Compositions may be used alone or may be blended with other polymers to obtain compositions having additional desired properties. The ratio of the ether component to the lactone is about 2:1.

Compositions of the present disclosure may possess adhesive forces of from about 5 g/cm² to about 80 g/cm², in embodiments from about 12 g/cm² to about 74 g/cm², in embodiments from about 20 g/cm² to about 70 g/cm². In embodiments, compositions of the present disclosure may have a weight average molecular weight from about 18000 g/mol to about 35000 g/mol, in embodiments from about 20000 g/mol to about 30000 g/mol.

In embodiments, the composition used to form a coating of the present disclosure may be functionalized with groups that assist in its binding to tissue. In embodiments, functional groups like hydroxyl, amine, sulfonate and carboxylate, may be added to the coating. For example, an N-hydroxysuccinimide ("NHS") ester may be used to functionalize the coating composition, which is reactive with amine groups present in tissue.

In embodiments, compositions of the present disclosure may be functionalized to possess electrophilic groups capable of reacting with nucleophiles to form covalent bonds. Covalent crosslinks or bonds refer to chemical groups formed by reaction of electrophiles with nucleophiles, that serve to covalently bind the composition tissue.

In embodiments the composition may be multifunctional, meaning that free ends of the composition, for example the ends of any arms on a multi-arm first component, may include electrophilic groups, such that, for example, an electrophilic functional group on the composition, in embodiments at a free end of the composition or any arm utilized as the first component, may react with a nucleophilic group on tissue to form a covalent bond.

Thus, for example, if the composition has electrophilic functional groups such as N-hydroxysuccinimides, it can react with nucleophilic functional groups in tissue, such as amines.

In embodiments, a multifunctional electrophilic polymer such as a multi-arm PEG transesterified with the second component, and further functionalized with multiple NHS groups, may be used as the coating.

In embodiments, the desired coating may have some solubility in a solvent which is a non-solvent for the material(s) forming the medical device, in embodiments a hernia patch. In general, the coating is applied to the patch by dissolving the coating in a solvent which is a non-solvent for the patch, and then dipping the patch into the solution. Illustrative of useful solvents is dimethyl sulfoxide (DMSO), which will dissolve the materials which form the coating but not the materials which form the patch.

Where utilized, the inclusion of the multi-arm PEG produces a very hydrophilic composition. The coating is stable when dry but becomes very tacky in the presence of water. The coating may be melt pressed onto a substrate, such as a mesh used to form a hernia patch. It is also soluble in solvents such as methylene chloride, so it may be sprayed directly onto tissue and/or onto a substrate to produce a thin coating layer on the substrate. Methods for applying a composition of the present disclosure to tissue and/or a substrate include dipping, spraying, solution casting, melt pressing, plasma deposition, combinations thereof, and the like.

Changing the ratio of the first component to the second component may alter solubility, as a greater amount of PEG will produce a coating with greater solubility in water.

Illustrative of useful devices which may be coated with the compositions of the present disclosure are hernia patches; tissue scaffolds; burn dressings; sponges; combinations thereof, and the like. The medical device is formed of a substrate, with the composition of the present disclosure forming a coating on at least a portion of a surface of the substrate.

In embodiments, the composition of the present disclosure may be utilized to form a thin polymer film, which may be used by itself as a tissue adhesive or sealant, or as a coating on a device to patch a defect, in embodiments a hernia, in vivo.

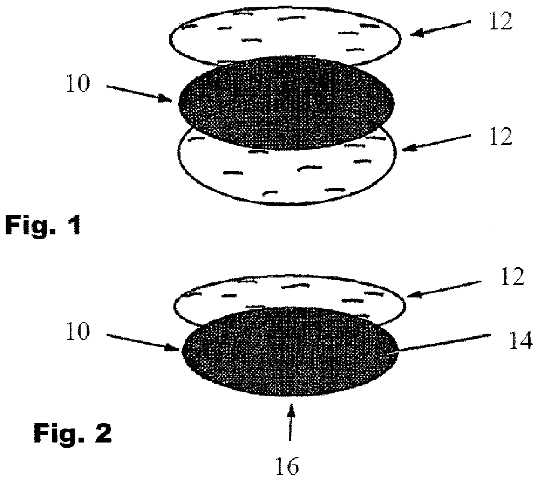

Turning now to the figures, a medical device having a composition of the present disclosure as a coating thereon is depicted. Referring to Figures 1 and 2, the medical device, in this case a hernia patch formed of a mesh substrate 10, includes a coating 12. The coating 12 includes a composition of the present disclosure. The coating layer may have a thickness greater than that of the strands of the mesh substrate 10. For example, the thickness of the layer of coating material may be about 1 mm to about 2 mm. The strands of the mesh substrate 10 may be entirely embedded in the bioactive coating 12 such that the outer surface of the mesh substrate 10 is covered entirely by the bioactive coating 12. In effect, the entire surgical implant may be encased in the bioactive coating as shown in Figure 1.

Thus, the surgical implant has no gaps or holes on its surface. This has the advantage of reducing the likelihood of bacteria becoming lodged on the strands of the mesh substrate 10 before implantation of the mesh substrate 10. Furthermore, the bioactive coating 12 makes the mesh substrate 10 more substantial and less flexible such that it is more easily handled by a surgeon. This is particularly useful when it is desired to place the mesh in a desired location in a conventional, open surgical procedure.

In an alternate embodiment shown in Figure 2, the bioactive coating 12 includes a layer of coating material applied to one face 14 of the mesh substrate 10, such that the mesh substrate has a first face 14 on which the coating material has been applied and a second face 16 on which the coating material has not been applied. Thus, the first and second faces 14 and 16 each have different characteristics. In use, the first face 14 possessing the composition of the present disclosure as coating 12 will be the face applied to tissue for affixing mesh substrate 10 to the tissue.

In embodiments, compositions of the present disclosure may be utilized to fix devices to tissue without the need for any additional fixation device, i.e., they may fix the device to tissue on their own. In embodiments, compositions of the present disclosure may be used to temporarily fix medical devices to tissue, allowing proper positioning of the device prior to final placement. As noted above, the composition of the present disclosure may affix the device to tissue without additional fixation devices, or application of an additional fixation device, such as a suture, a staple, a clip, a screw, a tack, an adhesive, a glue or sealant, combinations thereof, and the like, may be used to finally fix the device to tissue.

For example, the composition should be strong enough to provide at least temporary fixation of a medical device to tissue and, in some cases, removal from the tissue and re-adherence to the tissue as the medical device, in embodiments a hernia mesh, is repositioned to an optimal location by a medical professional applying the device to tissue. In this manner, for those cases where the medical device, such as a hernia patch, is applied to tissue, temporary fixation will allow the medical professional to continue with the procedure prior to final fixation of the device, and remove and re-attach the device, if necessary, if a more optimal location for the device becomes apparent during the procedure. Once the optimal location is determined, the composition of the present disclosure may finally fix the device to tissue, or additional fixation devices may be used to affix the device to tissue.

In another embodiment, the patch of the present disclosure may be secured to tissue using an additional fixation device, for example, a surgical fastener such as a surgical tack. Other surgical fasteners which may be used are within the purview of those skilled in the art, including staples, clips, helical fasteners, combinations thereof, and the like.

In embodiments, it may be advantageous to use surgical tacks as a surgical fastener to secure the patch to tissue. Tacks are known to resist larger removal forces compared with other fasteners. In addition, tacks only create one puncture, as compared to the multiple punctures created by staples. Tacks can also be used from only one side of the repair site, unlike staples, clips or other fasteners which require access to both sides of the repair site. This may be especially useful in the repair of a vaginal prolapse, where accessing the prolapse is difficult enough without having to access both sides of the prolapse. Suitable tacks which may be utilized to secure the patch of the present disclosure to tissue include, but are not limited to, the tacks described in U.S. Patent Application Publication No. 2004/0204723.

Suitable structures for other fasteners which may be utilized in conjunction with the patch of the present disclosure to secure same to tissue are within the purview of those skilled in the art and can include, for example, the suture anchor disclosed in U.S. Patent No. 5,964,78 3. Additional fasteners which may be utilized and tools for their insertion include the helical fasteners disclosed in U.S. Patent No. 6,562,051 and the screw fasteners disclosed in International Patent Application Publication No. WO 2004112841.

Surgical fasteners useful with a medical device, in embodiments a patch, may be made from bioabsorbable materials, non-bioabsorbable materials, and combinations thereof. Suitable materials which may be utilized include those described in U.S. Patent Application Publication No. 2004/0204723 and International Patent Application Publication No. WO 2004112841. Examples of absorbable materials which may be utilized include trimethylene carbonate, caprolactone, dioxanone, glycolic acid, lactic acid, glycolide, lactide, homopolymers thereof, copolymers thereof, and combinations thereof. Examples of non-absorbable materials which may be utilized include stainless steel, titanium, nickel, chrome alloys, and other biocompatible implantable metals. In embodiments, a shape memory alloy may be utilized as a fastener. Suitable shape memory materials include nitinol.

Surgical fasteners utilized in accordance with the present disclosure may be made into any size or shape to enhance their use depending on the size, shape and type of tissue located at the repair site.

Where used, surgical fasteners may be attached to a device, in embodiments a patch, in various ways. In embodiments, the ends of the patch may be directly attached to the fastener(s). In other embodiments, the patch may be curled around the fastener(s) prior to implantation. In yet another embodiment, the fastener may be placed inside the outer edge of the patch and implanted in a manner which pinches the patch up against the fastener and into the site of the injury.

In embodiments, any combination of the foregoing fasteners may be utilized to affix a medical device to tissue.

In some embodiments, the compositions of the present disclosure may be combined with one or more bioactive agents. The bioactive agent may be incorporated within the first component, incorporated within the second component, or both. Alternatively, the bioactive agent can be mixed with a coating including the first component and the second component prior to use. The term "bioactive agent", as used herein, is used in its broadest sense and includes any substance or mixture of substances that may have clinical use. Consequently, bioactive agents may or may not have pharmacological activity per se, e.g., a dye. Alternatively, a bioactive agent could be any agent which provides a therapeutic or prophylactic effect, a compound that affects or participates in tissue growth, cell growth or cell differentiation, a compound that may be able to invoke a biological action such as an immune response, or could play any other role in one or more biological processes.

Examples of classes of bioactive agents which may be utilized in accordance with the present disclosure include antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of bioactive agents may be used in the present compositions.

Suitable antimicrobial agents which may be included as a bioactive agent in the compositions of the present disclosure include triclosan, also known as 2,4,4'-trichloro-2'-hydroxydiphenyl ether; chlorhexidine and its salts, including chlorhexidine acetate, chlorhexidine gluconate, chlorhexidine hydrochloride, and chlorhexidine sulfate; silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin; tetracycline; aminoglycosides such as tobramycin and gentamicin; rifampicin; bacitracin; neomycin; chloramphenicol; miconazole; quinolones such as oxolinic acid, norfloxacin, nalidixic acid, pefloxacin, enoxacin and ciprofloxacin; penicillins such as oxacillin and pipracil; nonoxynol 9; fusidic acid; cephalosporins; and combinations thereof. In addition, antimicrobial proteins and peptides such as bovine lactoferrin and lactoferricin B may be included as a bioactive agent in the compositions of the present disclosure.

Other bioactive agents which may be included as a bioactive agent in the compositions of the present disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

A single bioactive agent may be utilized in the present compositions or, in alternate embodiments, any combination of bioactive agents may be utilized.

A variety of optional ingredients may also be added to the compositions of the present disclosure. For example, a phospholipid surfactant that provides antibacterial stabilizing properties and helps dispense other materials in the compositions may be added to the compositions of the present disclosure. Imaging agents such as iodine or barium sulfate, or fluorine, can also be combined with the compositions of the present disclosure to allow visualization of the surgical area through the use of imaging equipment, including X-ray, MRI, and CAT scan.

As noted above, in embodiments, the compositions of the present disclosure may be utilized as a coating on a hernia mesh or patch. While surgery to repair hernias has many differences in technique, anatomy and materials used, one similarity remains in the abdominal space: all patients require their implanted mesh to be mechanically fixed during ventral hernia repair. As there are many different types of mesh for hernia repair, there are also many forms of fixation which are used around the world. A hernia mesh that clings or temporarily adheres to the abdominal wall with a "post-it" effect makes positioning and fixation quick and simple. The coating of the present disclosure does not stick in a dry environment; however, it becomes exceptionally tacky after hydration. Application of this coating to mesh does not compromise the integrity, weight, or cost of the device while still providing enough adhesion and flexibility so that the mesh conforms to tissue while supporting its own weight.

The following Examples are being submitted to illustrate embodiments of the present disclosure. These Examples are intended to be illustrative only and are not intended to limit the scope of the present disclosure. Also, parts and percentages are by weight unless otherwise indicated. As used herein, "room temperature" refers to a temperature of from about 20°C to about 30° C.

### EXAMPLE 1

Compositions of the present disclosure were prepared as follows. A 4 arm PEG having a weight average molecular weight of about 10,000 Daltons was used as the first component. A synthetic polyester, which was a random copolymer possessing 17% caprolactone, 7% lactide, 7% trimethylene carbonate, and 69% glycolide (commercially available as POLYGLYTONE™ 6211 copolymer (hereinafter referred to as "POLYGLYTONE")) was used as the second component.

POLYGLYTONE was chosen for its relatively fast degradation profile as well as the presence of caprolactone, which contributes to polymer flexibility.

The targeted formulation was a polymer to be used as a tacky, fast absorbing coating to allow temporary fixation of hernia mesh. POLYGLYTONE may be formed into a thin film with very good flexibility. In order to take advantage of this flexibility, the incorporation of polyethylene glycol adds a hydrophilic component to POLYGLYTONE which by itself is hydrophobic. Another property targeted by the material is a fast mass resorption rate to allow tissue integration into the hernia mesh. The degradation profile of POLYGLYTONE in an in-vitro bath at 37°C was nearly 60 days. The addition of PEG resulted in an acceleration of hydrolysis and a reduced degradation profile.

After initial testing with the 4 arm PEG blended with POLYGLYTONE, an optimal ratio of 2:1 PEG to POLYGLYTONE was used for additional testing. Although in vitro tests were not monitored to completion on these early samples, it was apparent that there were little changes in the first 2 weeks. This most likely was due to the PEG star molecules undergoing transesterification but also leading to crosslinking. The material was
67% 4arm PEG10,000 / 33% POLYGLYTONE
mixed in a traditional heating mantle at 165°C.

Although this product had a very tacky feel upon hydration, concerns existed about extending the persistence through crosslinking, which led to the use of straight chain PEG molecules. PEG 1000 blends with POLYGLYTONE created liquid final products, while PEG 3400 blended with POLYGLYTONE took a long time to solidify. PEG 4600 blended with POLYGLYTONE generated a very good material. A small percentage of PEG1000 was included in subsequent formulations to soften the material even more. This blend was as follows:
67% PEG (96% PEG4,600/4% PEG1000) / 33% POLYGLYTONE

Size exclusion chromatography was used to conform the existence of the new material. Size exclusion chromatography separates polymer chains of varying size by passing them over beads containing micron size pores. As the sample makes its way through a column, the smaller molecules spend more time travelling in these void spaces than the larger ones. A higher molecular weight molecule such as POLYGLYTONE elutes through the system faster than the transesterified samples, which were modified to lower molecular weights. The results demonstrated that blends with higher ratios of POLYGLYTONE eluted faster than the blends with lower ratios of POLYGLYTONE.

Higher levels of the lower molecular weight PEG also led to a formulation which broke down rapidly.

### EXAMPLE 2

Samples of the optimal formulation of Example 1,67% PEG (96% PEG 4,600/4% PEG 1000) / 33% POLYGLYTONE were placed on a mesh.

Films of uniform thickness as listed in table 1 below were prepared by melting individual samples on PTFE sheets and compressing into a flat sheet. A 1 inch diameter circular sample was punched from the newly formed sheet. The circles were added to a falcon tube which was then filled with 40 ml of pH 7.4 phosphate buffered saline (PBS) buffer and placed in a 37°C bath. The falcon tubes were monitored to observe changes.

**Table 1**

| Film | Thickness (in) |
|---|---|
| A | 0.0075 |
| B | 0.0051 |
| C | 0.0058 |
| D | 0.0066 |

Differential Scanning Calorimeters (DSC) measure temperatures and heat flow associated with thermal transitions in a material. A TA Instruments Q100 DSC instrument was used. The thermal properties of the blended product changed from original POLYGLYTONE. Due to the low crystallinity of the material, an annealing hold step of 60 minutes at 0°C was included. The results showed a Tₘ of 46°C displayed on the second heat with a T_{g} of -23°C. This is a dramatic change from the DSC of POLYGLYTONE by itself, which had a typical Tₘ of 135° C and T_{g} of 20°C.

**Table 2**

| Sample ID | Second Heat | | |
|---|---|---|---|
| | T_{g} (°C) | Tₘ (°C) | Tₘ ΔH (J/g) |
| PEG 60/POLYGLYTONE 40 (High POLYGLYTONE) | -22.95 | 46.24 | 62.35 |
| PEG 67/POLYGLYTONE 33 (mid POLYGLYTONE) | -21.60 | 47.30 | 79.24 |
| PEG 67/POLYGLYTONE 33 (mid-POLYGLYTONE) | -13.93 | 47.88 | 79.65 |
| PEG67/POLYGLYTONE 33 (mid-POLYGLYTONE) | -12.76 | 47.98 | 81.04 |
| PEG 75/POLYGLYTONE 25 (Low POLYGLYTONE) | -23.80 | 48.42 | 96.21 |
| POLYGLYTONE | 16 | 14 | 5.7 |

Individual modified lots in Table 2 showed similar thermal profiles when compared to one another but clearly different from POLYGLYTONE.

The blending of polyethylene glycols with POLYGLYTONE via transesterification resulted in a flexible coating that could be handled easily at room temperature and became tacky upon exposure to moisture. Analytical techniques such as multi angle light scattering and differential scanning calorimetry were used to determine variations in the final product.

### EXAMPLE 3

Tacky mesh samples were prepared and tested as follows:

**Table 3**

| Qty | Material |
|---|---|
| 1 | 8.8cm x 14cm Composite Mesh laminated with 25/75 POLYGLYTONE /PEG (96:4 PEG 4600/PEG 1000) |
| 1 | 8.8cm x 14cm Composite Mesh laminated with 33/67 POLYGLYTONE /PEG (96:4 PEG 4600/PEG 1000) |
| 1 | 8.8cm x 14cm Composite Mesh laminated with 40/60 POLYGLYTONE /PEG (99:1 PEG 4600/PEG 1000) |
| 1 | 8.8cm x 14cm Composite Mesh (Control) |

The composite mesh was a polypropylene mesh bonded to a film of POLYGLYTONE with polycaprolactone having a molecular weight of about 80,000. Each 8.8 cm x 14 cm piece of composite mesh was cut into ten 2.5 cm x 2.5 cm segments. The adhesive forces for each formulation (n=10) were measured using a TA.XT Plus Texture Analyzer.

The tack test revealed significant differences in adhesive forces between formulations. This test was performed using a probe with a 1cm diameter. The results for each formulation are listed in the tables below.

**Table 4**

| Composite Mesh laminated with 25/75 POLYGLYTONE/PEG (96:4 PEG 4600/PEG 1000) | |
|---|---|
| Sample | Tack Force (g) |
| 1 | 6.956 |
| 2 | 8.237 |
| 3 | 8.424 |
| 4 | 9.804 |
| 5 | 6.956 |
| 6 | 11.075 |
| 7 | 9.946 |
| 8 | 9.41 |
| 9 | 8.566 |
| 10 | 6.551 |
| Average | 8.592 |
| Standard Deviation | 1.484 |
| Coefficient of Variation | 17.267 |

**Table 5**

| Composite Mesh laminated with 33/67 POLYGLYTONE/PEG (96:4 PEG 4600/PEG 1000) | |
|---|---|
| Sample | Tack Force (g) |
| 11 | 15.489 |
| 12 | 23.398 |
| 13 | 29.237 |
| 14 | 23.267 |
| 15 | 21.7 |
| 16 | 24.691 |
| 17 | 25.501 |
| 18 | 21.689 |
| 19 | 16.968 |
| 20 | 18.907 |
| Average | 22.0847 |
| Standard Deviation | 4.121 |
| Coefficient of Variation | 18.659 |

**Table 6**

| Composite Mesh laminated with 40/60 POLYGLYTONE/PEG (99:1 PEG 4600/PEG 1000) | |
|---|---|
| Sample | Tack Force (g) |
| 21 | 4.502 |
| 22 | 13.736 |
| 23 | 4.415 |
| 24 | 5.477 |
| 25 | 9.705 |
| 26 | 7.613 |
| 27 | 6.781 |
| 28 | 7.712 |
| 29 | 5.148 |
| 30 | 7.909 |
| Average | 7.3 |
| Standard Deviation | 2.833 |
| Coefficient of Variation | 38.808 |

Composite Mesh coated with transesterified POLYGLYTONE and low molecular weight PEG blends showed promising initial results on the bench-top. It was presumed that higher levels of POLYGLYTONE would prolong the persistence of the material in vivo. Effort was taken to limit the persistence of the "tacky" coating while maintaining the adhesive forces required to achieve the "post-it" effect.

The table below summarizes the average adhesive forces observed (g/cm²) for each formulation in comparison to Composite mesh alone.

**Table 7**

| Formulation | Average Tack Force (g/cm²) |
|---|---|
| Composite Mesh Control | 0.3 |
| 25/75 POLYGLYTONE /PEG (96:4 PEG 4600/PEG 1000) | 10.9 |
| 33/67 POLYGLYTONE /PEG (96:4 PEG 4600/PEG 1000) | 28.1 |
| 40/60 POLYGLYTONE /PEG (99:1 PEG 4600/PEG 1000) | 9.3 |

The mechanical testing results for this study demonstrated that the addition of a thin layer of transesterified POLYGLYTONE/PEG blend to Composite Mesh significantly enhanced the adhesive attraction of the implant to the peritoneum. The weight of a hydrated, coated piece of mesh was 0.025 g /cm². In reference to the weight of the coated mesh, all three formulations sufficiently held the mesh. The formulation having the best average tack force contained 33% POLYGLYTONE. This formulation saw the maximum adhesive forces with the minimum amount of POLYGLYTONE required to reach these forces.

In this study, the transesterified polymer blend of 33% POLYGLYTONE /67% PEG (96:4 PEG 4600/PEG 1000) showed the maximum adhesive forces.

### EXAMPLE 4

Hydrogels were prepared as described above in Example 1, wherein the formulation was 67% PEG/33% POLYGLYTONE. The PEG component was a combination of a linear PEG and a 4-armed PEG.

The reaction materials, and their sources, are set forth below in Table 8.

**Table 8**

| Reaction Materials | Manufacturer |
|---|---|
| Polyethylene glycol, Mn=1,000 | Sigma Aldrich |
| Polyethylene glycol, Mn=4,600 | Sigma Aldrich |
| Polyethylene glycol, Mn=8,000 | Sigma Aldrich |
| Polyethylene glycol, Mn=10,000 | Sigma Aldrich |
| 4arm Polyethylene glycol, Mn=2,000 | KemTech |
| 4arm Polyethylene glycol, Mn=10,000 | KemTech |
| POLYGLYTONE | Covidien |
| Catalyst, Stannous Octoate | Brand-Nu Labs |

Various ratios of the PEG were used to produce adhesive compositions in accordance with the present disclosure. The components used to form the various compositions are set forth below in Tables 9-21.

**Table 9**

| keaction Batch [PEG 99:01] | Mass (g) | Ratio |
|---|---|---|
| Major PEG Component | 66.0g | 67% [99:01] |
| Minor PEG Component | 0.675g | |
| POLYGLYTONE | 33.25g | 33% |
| Stannous Octoate | 0.075g | 0.075% |

**Table 10**

| keaction Batch [PEG 90:10] | Mass (g) | Ratio |
|---|---|---|
| Major PEG Component | 60.0g | 57% [90:10] |
| Minor PEG Component | 6.675g | |
| POLYGLYTONE | 33.25g | 33% |
| Stannous Octoate | 0.075g | 0.075% |

**Table 11**

| Reaction Batch [PEG 80:20] | Mass (g) | Ratio |
|---|---|---|
| Major PEG Component | 53.34g | 67% [80:20] |
| Minor PEG Component | 13.335g | |
| POLYGLYTONE | 33.25g | 33% |
| Stannous Octoate | 0.075g | 0.075% |

**Table 12**

| Reaction Batch [PEG 97:03] | Mass (g) | Ratio |
|---|---|---|
| Major PEG Component | 64.675g | 67% [97:03] |
| Minor PEG Component | 2.0g | |
| POLYGLYTONE | 33.25g | 33% |
| Stannous Octoate | 0.075g | 0.075% |

**Table 13**

| Reaction Batch [PEG 96:04] | Mass (g) | Ratio |
|---|---|---|
| Major PEG Component | 64.008g | 67% [96:04] |
| Minor PEG Component | 2.667g | |
| POLYGLYTONE | 33.25g | 33% |
| Stannous Octoate | 0.075g | 0.075% |

**Table 14**

| Reaction Batch [PEG 95:05] | Mass (g) | Ratio |
|---|---|---|
| Major PEG Component | 63.34g | 67% [95:05] |
| Minor PEG Component | 3.335g | |
| POLYGLYTONE | 33.25g | 33% |
| Stannous Octoate | 0.075g | 0.075% |

**Table 15**

| Reaction Batch [PEG 94:06] | Mass (g) | Ratio |
|---|---|---|
| Major PEG Component | 62.675g | 67% [94:06] |
| Minor PEG Component | 4.0g | |
| POLYGLYTONE | 33.25g | 33% |
| Stannous Octoate | 0.075g | 0.075% |

**Table 16**

| keaction Batch [PEG 92:08] | Mass (g) | Ratio |
|---|---|---|
| Major PEG Component | 61.341g | 67% [92:08] |
| Minor PEG Component | 5.334g | |
| POLYGLYTONE | 33.25g | 33% |
| Stannous Octoate | 0.075g | 0.075% |

**Table 17**

| keaction Batch [PEG 83:15] | Mass (g) | Ratio |
|---|---|---|
| Major PEG Component | 56.675g | 67% [85:15] |
| Minor PEG Component | 10.0g | |
| POLYGLYTONE | 33.25g | 33% |
| Stannous Octoate | 0.075g | 0.075% |

**Table 18**

| keaction Batch [PEG 7:23] | Mass (g) | Ratio |
|---|---|---|
| Major PEG Component | 51.34 g | 67% [77:23] |
| Minor PEG Component | 15.335g | |
| POLYGLYTONE | 33.25g | 33% |
| Stannous Octoate | 0.075g | 0.075% |

**Table 19**

| Reaction Batch [PEG 75:25] | Mass (g) | Ratio |
|---|---|---|
| Major PEG Component | 50.01g | 67% [75:25] |
| Minor PEG Component | 16.665g | |
| POLYGLYTONE | 33.25g | 33% |
| Stannous Octoate | 0.075g | 0.075% |

**Table 20**

| keaction Batch [PEG 70:30] | Mass (g) | Ratio |
|---|---|---|
| Major PEG Component | 46.6725g | 67% [70:30] |
| Minor PEG Component | 20.0025g | |
| POLYGLYTONE | 33.25g | 33% |
| Stannous Octoate | 0.075g | 0.075% |

**Table 21**

| keaction Batch [PEG 65:35] | Mass (g) | Ratio |
|---|---|---|
| Major PEG Component | 43.3388g | 67% [65:35] |
| Minor PEG Component | 23.3363g | |
| POLYGLYTONE | 33.25g | 33% |
| Stannous Octoate | 0.075g | 0.075% |

Adhesive compositions were prepared as follows. The reaction components were added to a reaction vessel in a dry lab and sealed with a polished stir shaft/glass adapter, rubber septum, and polytetrafluoroethylene (PTFE) stopper. In a fume hood, the flask was added to a heating block and secured to an overhead stirrer. A rubber septum was replaced by a dual hose connection adapter and the nitrogen hose was hooked up to one side of the adapter. The opposing side of the dual adapter was connected to a mineral oil bubbler before turning on the nitrogen flow. To initiate the drying process, a hot plate was set to 110°C and the stirrer to 50 revolutions per minute (rpm). After 30 minutes of mixing, the PEG content of the vessel was melted. At this time, the PTFE stopper was replaced with a rubber septum. A glass pipette was then passed through the rubber septum and positioned with the tip just below the surface of the molten PEG. The nitrogen was then connected to the pipette end and the flow adjusted so that there was steady bubbling under the PEG surface. To redirect the nitrogen flow out towards the bubbler, a plug was placed over the open side of the dual hose connection adapter. The reaction contents are allowed to dry overnight under these conditions.

After drying overnight, the reaction was set up to run by replacing the rubber septum and pipette with the PTFE stopper. The nitrogen hose was then reattached to the dual hose connection adapter to place the flask contents back under nitrogen. The reaction was initiated by ramping up the temperature to 185°C and the stirring to 300 rpm. After approximately 1 hour, the reaction temperature was reduced to 145°C and set to mix at 150 rpm. This was done to completely melt the POLYGLYTONE component which has a high melting point of 125-145°C. The reaction was allowed to run overnight for approximately 18 hours. The molten product was poured into a clean Teflon dish and allowed to fully solidify in the ventilation hood.

The adhesives prepared above were then subjected to a tack test as described above in Example 3. The tack test results for all formulations with a major PEG component of 4600 MW are summarized in the table below.

**Table 22**

| POLYGLYTONE/PEG | Major PEG | Minor PEG | PEG Ratio | Avg. Adhesive Force (g/cm) | Std. Dev | Coef. Of Variation |
|---|---|---|---|---|---|---|
| 33/67 | 4600 | 1000 | 99:01 | 22.38 | 11.14 | 49.75 |
| 33/67 | 4600 | 1000 | 90:10 | 22.38 | 15.73 | 52.19 |
| 33/67 | 4600 | 1000 | 80:20 | 24.74 | 11.11 | 44.92 |
| 33/67 | 4600 | 4arm2k | 99:01 | 73.99 | 26.18 | 35.38 |
| 33/67 | 4600 | 4arm2k | 90:10 | 13.23 | 6.21 | 46.96 |
| 33/67 | 4600 | 4arm2k | 80:20 | 44.62 | 12.43 | 27.87 |
| 33/67 | 4600 | 8000 | 99:01 | 24.88 | 7.36 | 29.60 |
| 33/67 | 4600 | 8000 | 90:10 | 19.45 | 7.09 | 36.43 |
| 33/67 | 4600 | 8000 | 80:20 | 18.77 | 7.70 | 41.02 |
| 33/67 | 4600 | 4arm10k | 99:01 | 18.98 | 5.69 | 29.97 |
| 33/67 | 4600 | 4arm10k | 90:10 | 21.53 | 8.61 | 39.97 |
| 33/67 | 4600 | 4arm10k | 80:20 | 21.88 | 10.69 | 48.83 |

The PEGs in the above tables, as well as the following tables, were linear, except where "arm" is present in the description of the PEG. The numbers under the "Major PEG" and "Minor PEG" headings are the weight average molecular weights (MW) of the PEGs, with "k" designating thousands.

All formulations except for 33% POLYGLYTONE/ 67% PEG (90:10 4600/4arm2k) and 33% POLYGLYTONE/67% PEG (80:20 4600/8000) cleared the Lower Specification Limit (LSL) of 15 g/cm². The LSL is the average adhesive force observed for the optimal tacky mesh formulation and was considered the lowest acceptable adhesive force for a self-fixating hernia mesh. The highest performing formulation of this subset was 33%POLYGLYTONE/67% PEG (99:01 4600/4arm2k) with an average adhesive force of 73.99g/cm², followed by 33% POLYGLYTONE/67% PEG (80:20 4600/4arm2k) at 44.62 g/cm². All other formulations that cleared the LSL had average adhesive forces in the range of 18.98 g/cm² to 24.88 g/cm². Of these formulations, 33% POLYGLYTONE/67% PEG (80:20 4600/1000) was optimized based on tactile feel of the polymer in addition to the highest performing formulations.

The tack test results for all formulations with a major PEG component of 8000MW are summarized in the table below.

**Table 23**

| POLYGLYTONE/PEG | Major PEG | Minor PEG | PEG Ratio | Avg. Adhesive Force (g/cm²) | Std. Dev | Coef. Of Variation |
|---|---|---|---|---|---|---|
| 33/67 | 8000 | 1000 | 99:01 | 12.66 | 6.64 | 52.46 |
| 33/67 | 8000 | 1000 | 90:10 | 13.86 | 4.95 | 35.73 |
| 33/67 | 8000 | 1000 | 80:20 | 30.29 | 23.57 | 77.81 |
| 33/67 | 8000 | 4arm2k | 99:01 | 10.55 | 3.74 | 35.43 |
| 33/67 | 8000 | 4arm2k | 90:10 | 21.32 | 5.54 | 25.96 |
| 33/67 | 8000 | 4arm2k | 80:20 | 28.63 | 13.67 | 47.75 |
| 33/67 | 8000 | 4600 | 99:01 | 11.42 | 4.97 | 43.58 |
| 33/67 | 8000 | 4600 | 90:10 | 9.42 | 4.82 | 51.12 |
| 33/67 | 8000 | 4600 | 80:20 | 18.29 | 8.91 | 48.72 |
| 33/67 | 8000 | 10000 | 99:01 | 15.89 | 5.19 | 32.67 |
| 33/67 | 8000 | 10000 | 90:10 | 17.91 | 6.02 | 33.61 |
| 33/67 | 8000 | 10000 | 80:20 | 17.53 | 5.88 | 33.56 |
| 33/67 | 8000 | 4arm10k | 99:01 | 22.40 | 12.21 | 54.49 |
| 33/67 | 8000 | 4arm10k | 90:10 | 13.54 | 6.30 | 46.53 |
| 33/67 | 8000 | 4arm10k | 80:20 | 20.28 | 11.00 | 54.25 |

The majority of the formulations failed to clear the Lower Specification Limit (LSL) of 15 g/cm². The highest performing formulation of this subset was 33%POLYGLYTONE/67% PEG (80:20 8000/1000) with an average adhesive force of 30.29g/cm². All other formulations that cleared the LSL had average adhesive forces in the range of 17.53 g/cm² to 28.63 g/cm².

The tack test results for all formulations with a major PEG component of 10,000MW are summarized in the table below.

**Table 24**

| POLYGLYTONE/PEG | Major PEG | Minor PEG | PEG Ratio | Avg. Adhesive Force (g/cm²) | Std. Dev | Coef. Of Variation |
|---|---|---|---|---|---|---|
| 33/67 | 10,000 | 1000 | 99:01 | 10.21 | 3.55 | 34.81 |
| 33/67 | 10,000 | 1000 | 90:10 | 15.21 | 7.14 | 46.92 |
| 33/67 | 10,000 | 1000 | 80:20 | 33.27 | 15.83 | 47.56 |
| 33/67 | 10,000 | 4arm2k | 99:01 | 15.07 | 6.85 | 45.43 |
| 33/67 | 10,000 | 4arm2k | 90:10 | 15.62 | 7.88 | 50.47 |
| 33/67 | 10,000 | 4arm2k | 80:20 | 19.48 | 7.72 | 39.61 |
| 33/67 | 10,000 | 4600 | 99:01 | 7.77 | 2.67 | 34.40 |
| 33/67 | 10,000 | 4600 | 90:10 | 8.09 | 2.89 | 35.71 |
| 33/67 | 10,000 | 4600 | 80:20 | 8.77 | 3.68 | 41.97 |
| 33/67 | 10,000 | 8000 | 99:01 | 14.91 | 6.76 | 45.35 |
| 33/67 | 10,000 | 8000 | 90:10 | 14.88 | 6.76 | 45.43 |
| 33/67 | 10,000 | 8000 | 80:20 | 13.35 | 3.52 | 26.38 |
| 33/67 | 10,000 | 4arm10k | 99:01 | 14.55 | 7.63 | 52.42 |
| 33/67 | 10,000 | 4arm10k | 90:10 | 14.55 | 7.23 | 49.73 |
| 33/67 | 10,000 | 4arm10k | 80:20 | 14.55 | 6.90 | 47.42 |

The majority of the formulations failed to clear the Lower Specification Limit (LSL) of 15 g/cm². The highest performing formulation of this subset was 33%POLYGLYTONE/67% PEG (80:20 10,000/1000) with an average adhesive force of 33.27 g/cm². The only additional formulation that cleared the LSL was 33% POLYGLYTONE/ 67% PEG (80:20 10,000/4arm2k) with an average adhesive force of 19.48 g/cm².

The tack test results for all formulations with a major PEG component of 4-arm 10,000MW are summarized in the table below.

**Table 25**

| POLYGLYTONE/PEG | Major PEG | Minor PEG | PEG Ratio | Avg. Adhesive Force (g/cm²) | Std. Dev | Coef. Of Variation |
|---|---|---|---|---|---|---|
| 33/67 | 4arm10k | 1000 | 99:01 | 22.20 | 8.69 | 39.13 |
| 33/67 | 4arm10k | 1000 | 90:10 | 33.75 | 13.20 | 39.10 |
| 33/67 | 4arm10k | 1000 | 80:20 | 38.28 | 11.97 | 31.27 |
| 33/67 | 4arm10k | 4arm2k | 99:01 | 12.41 | 7.76 | 62.55 |
| 33/67 | 4arm10k | 4arm2k | 90:10 | 22.72 | 7.36 | 32.40 |
| 33/67 | 4arm10k | 4arm2k | 80:20 | 29.47 | 12.01 | 40.74 |
| 33/67 | 4arm10k | 4600 | 99:01 | 22.36 | 5.15 | 23.01 |
| 33/67 | 4arm10k | 4600 | 90:10 | 30.46 | 7.66 | 25.16 |
| 33/67 | 4arm10k | 4600 | 80:20 | 28.91 | 10.28 | 35.56 |
| 33/67 | 4arm10k | 8000 | 99:01 | 26.33 | 10.89 | 41.35 |
| 33/67 | 4arm10k | 8000 | 90:10 | 26.30 | 10.94 | 41.60 |
| 33/67 | 4arm10k | 8000 | 80:20 | 23.57 | 8.39 | 35.60 |
| 33/67 | 4arm10k | 10000 | 99:01 | 15.37 | 3.55 | 23.10 |
| 33/67 | 4arm10k | 10000 | 90:10 | 16.21 | 6.92 | 42.68 |
| 33/67 | 4arm10k | 10000 | 80:20 | 12.27 | 3.05 | 24.83 |

All formulations except for 33% POLYGLYTONE/ 67% PEG (99:01 4arm10k/4arm2k) and 33% POLYGLYTONE/67% PEG (80:20 4arm10k/10,000) cleared the Lower Specification Limit (LSL) of 15 g/cm². The highest performing formulation of this subset was 33%POLYGLYTONE/67% PEG (80:20 4arm10k/1000) with an average adhesive force of 38.28g/cm², followed by 33% POLYGLYTONE/67% PEG (90:10 4arm10k/1000) at 33.75 g/cm², and 33% POLYGLYTONE/67% PEG (80:20 4arm10k/4arm2k) at 29.47 g/cm². All other formulations that cleared the LSL had average adhesive forces in the range of 15.37 g/cm² to 28.91 g/cm².

The tack test results for all ratios with a major PEG component of 4600MW and a minor PEG component of 1000MW are summarized in the table below.

**Table 26**

| POLYGLYTONE/PEG | Major PEG | Minor PEG | PEG Ratio | Avg. Adhesive Force (g/cm²) | Std. Dev. | Coef. Of Variation |
|---|---|---|---|---|---|---|
| 33/67 | 4600 | 1k | 99:01 | 22.38 | 11.14 | 49.78 |
| 33/67 | 4600 | 1k | 96:04 | 19.54 | 15.12 | 77.38 |
| 33/67 | 4600 | 1k | 94:06 | 50.24 | 22.85 | 45.48 |
| 33/67 | 4600 | 1k | 90:10 | 30.15 | 15.73 | 52.17 |
| 33/67 | 4600 | 1k | 80:20 | 28.65 | 3.73 | 13.02 |

All formulations except for 33% POLYGLYTONE/ 67% PEG (96:04 4600/1000) cleared the Lower Specification Limit (LSL) of 15 g/cm². The highest performing formulation of this subset was 33%POLYGLYTONE/67% PEG (94:06 4600/1000) with an average adhesive force of 50.24g/cm2.

The tack test results for all ratios with a major PEG component of 4600MW and a minor PEG component of 4-arm 2000MW are summarized in the table below.

**Table 27**

| POLYGLYTONE/PEG | Major PEG | Minor PEG | PEG Ratio | Avg. Adhesive Force (g/cm²) | Std. Dev. | Coef. Of Variation |
|---|---|---|---|---|---|---|
| 33/67 | 4600 | 4arm2k | 99:01 | 73.99 | 26.18 | 35.38 |
| 33/67 | 4600 | 4arm2k | 97:03 | 39.09 | 15.90 | 40.68 |
| 33/67 | 4600 | 4arm2k | 95:05 | 28.68 | 15.69 | 54.71 |
| 33/67 | 4600 | 4arm2k | 92:08 | 32.53 | 12.26 | 37.69 |
| 33/67 | 4600 | 4arm2k | 90:10 | 13.23 | 6.21 | 46.96 |
| 33/67 | 4600 | 4arm2k | 80:20 | 44.62 | 12.43 | 27.86 |

As these were single formulations, additional tests were conducted on formulations made from both the PEGs at 99:01 ratio (4600/4arm2k) and 90:10 ratio (4600/4arm2k). Compositions were prepared as outlined above. The results obtained for the 33% POLYGLYTONE/67% PEG (90:10 4600/4arm2k) are set forth below in Table 28.

**Table 28**

| Sample | Adhesive Force (g/cm²) |
|---|---|
| A | 61.60 |
| B | 106.30 |
| c | 69.08 |
| D | 71.53 |
| E | 56.52 |
| F | 156.25 |
| G | 131.01 |
| H | 94.64 |
| I | 68.92 |
| J | 128.58 |
| K | 77.47 |
| L | 112.77 |
| Average Force | 94.56 |
| Standard Deviation | 32.23 |
| Coefficient of Variation | 34.08 |

As can be seen from the above, the average adhesive force for these compositions was surprisingly high, at 94.56 g/cm².

Similar compositions were made for the formulations of 33% POLYGLYTONE/67% PEG (99:01 4600/4arm2k). The average adhesive force for these compositions was found to be 34.09 g/cm².

The tack test results for all ratios with a major PEG component of 8000MW and a minor PEG component of 1000MW are summarized in the table below.

**Table 29**

| POLYGLYTONE/PEG | Major PEG | Minor PEG | PEG Ratio | Avg. Adhesive Force (g/cm²) | Std. Dev. | Coef. Of Variation |
|---|---|---|---|---|---|---|
| 33/67 | 8k | 1k | 99:01 | 13.43 | 4.78 | 35.63 |
| 33/67 | 8k | 1k | 95:05 | 11.35 | 3.52 | 31.05 |
| 33/67 | 8k | 1k | 90:10 | 13.86 | 4.95 | 35.73 |
| 33/67 | 8k | 1k | 85:15 | 37.93 | 12.82 | 33.80 |
| 33/67 | 8k | 1k | 80:20 | 30.29 | 23.57 | 77.81 |
| 33/67 | 8k | 1k | 77:23 | 35.51 | 9.54 | 26.87 |
| 33/67 | 8k | 1k | 75:25 | 46.72 | 17.78 | 38.06 |
| 33/67 | 8k | 1k | 70:30 | 32.17 | 6.32 | 19.65 |
| 33/67 | 8k | 1k | 65:35 | 15.11 | 4.89 | 32.36 |

All ratios except for 99:01, 95:05, 90:10, and 65:35 cleared the Lower Specification Limit (LSL) of 15 g/cm². The highest performing formulation of this subset was 33%POLYGLYTONE/67% PEG (75:25 8000/1000) with an average adhesive force of 46.72 g/cm2.

The tack test results for all ratios with a major PEG component of 4-arm 10,000MW and a minor PEG component of 1000MW are summarized in the table below.

**Table 30**

| POLYGLYTONE/PEG | Major PEG | Minor PEG | PEG Ratio | Avg. Adhesive Force (g/cm²) | Std. Dev. | Coef. Of Variation |
|---|---|---|---|---|---|---|
| 33/67 | 4arm10k | 1k | 99:01 | 29.37 | 4.73 | 16.12 |
| 33/67 | 4arm10k | 1k | 95:05 | 19.31 | 10.56 | 54.72 |
| 33/67 | 4arm10k | 1k | 90:10 | 32.34 | 13.32 | 41.20 |
| 33/67 | 4arm10k | 1k | 85:15 | 51.74 | 17.20 | 33.24 |
| 33/67 | 4arm10k | 1k | 80:20 | 38.28 | 11.97 | 31.27 |
| 33/67 | 4arm10k | 1k | 75:25 | 40.09 | 6.43 | 16.03 |

All formulations except for 33% POLYGLYTONE/67% PEG (95:05 4arm10k/1000) cleared the Lower Specification Limit (LSL) of 15 g/cm2. The highest performing formulation of this subset was 33%POLYGLYTONE/67% PEG (85:15 4arm10k/1000) with an average adhesive force of 51.74 g/cm2.

The tack test results for all ratios with a major PEG component of 4-arm 10,000MW and a minor PEG component of 4-arm 2000MW are summarized in the table below.

**Table 31**

| POLYGLYTONE/PEG | Major PEG | Minor PEG | PEG Ratio | Avg. Adhesive Force (g/cm²) | Std. Dev. | Coef. Of Variation |
|---|---|---|---|---|---|---|
| 33/67 | 4arm10k | 4arm2k | 99:01 | 12.41 | 7.76 | 62.55 |
| 33/67 | 4arm10k | 4arm2k | 90:10 | 22.72 | 7.36 | 32.40 |
| 33/67 | 4arm10k | 4arm2k | 85:15 | 32.48 | 14.36 | 44.21 |
| 33/67 | 4arm10k | 4arm2k | 80:20 | 44.73 | 19.92 | 44.54 |
| 33/67 | 4arm10k | 4arm2k | 75:25 | 46.42 | 14.32 | 30.85 |
| 33/67 | 4arm10k | 4arm2k | 70:30 | 57.95 | 13.32 | 22.99 |

All formulations except for 33% POLYGLYTONE/67% PEG (99:01 4arm10k/4arm2k) cleared the Lower Specification Limit (LSL) of 15 g/cm². The highest performing formulation of this subset was 33%POLYGLYTONE/67% PEG (70:30 4arm10k/4arm2k) with an average adhesive force of 57.95 g/cm².

The tack test results for the five highest performing formulations are summarized in the table below.

**Table 32**

| POLYGLYTONE/PEG | Major PEG | Minor PEG | PEG Ratio | Avg. Adhesive Force (g/cm²) | Std. Dev. | Coef. Of Variation |
|---|---|---|---|---|---|---|
| 33/67 | 4600 | 1k | 94:06 | 50.24 | 22.85 | 45.48 |
| 33/67 | 4600 | 4arm2k | 99:01 | 73.99 | 26.18 | 35.38 |
| 33/67 | 8k | 1k | 75:25 | 46.72 | 17.78 | 38.06 |
| 33/67 | 4arm10k | 1k | 85:15 | 51.74 | 17.20 | 33.24 |
| 33/67 | 4arm10k | 4arm2k | 70:30 | 57.95 | 13.32 | 22.99 |
| 33/67 | 4600 | 1000 | 96:04 | 15.35 | 11.87 | 77.31 |

The highest performing formulation of all formulations evaluated in this study was 33%POLYGLYTONE/67% PEG (99:01 4600/4arm2k) with an average adhesive force of 73.99 g/cm2.

As is apparent from the above, the transesterified polymer blend of 33% POLYGLYTONE /67% PEG, where the PEG was 90% by weight of a linear PEG having a molecular weight of about 4,600 combined with 10% of a 4-armed PEG having a molecular weight of about 2000, showed the highest adhesive forces.

## Claims

1. A biocompatible adhesive comprising:
a first component including at least one linear polyethylene glycol having a molecular weight from 1,000 to 10,000 Daltons, in combination with at least one multi-arm polyethylene glycol having a molecular weight from 1,000 to 3,000 Daltons; and
a second component including a lactone.

2. The biocompatible adhesive of claim 1, wherein the first component includes the at least one linear polyethylene glycol and the at least one multi-arm polyethylene glycol at a ratio from 99:1 to 80:20 by weight.

3. The biocompatible adhesive of claim 2, wherein the first component includes the at least one linear polyethylene glycol and the at least one multi-arm polyethylene glycol at a ratio from 98:2 to 88:12 by weight.

4. The biocompatible adhesive of any preceding claim, wherein the multi-arm polyethylene glycol has from four to twelve arms.

5. The biocompatible adhesive of claim 4, wherein the multi-arm polyethylene glycol has four arms.

6. The biocompatible adhesive of claim 5, wherein the at least one linear polyethylene glycol has a molecular weight of about 4,600 Daltons and the at least one multi-arm polyethylene glycol has a molecular weight of about 2,000 Daltons.

7. The biocompatible adhesive of any preceding claim, wherein the lactone is selected from the group consisting of lactide, glycolide, caprolactones, valerolactones, dioxanones, dioxepanones, trimethylene carbonates, propiolactones, butyrolactone, and combinations thereof.

8. The biocompatible adhesive of any preceding claim, wherein the lactone is a quaternary polymer including from 62% to 72% by weight glycolide, from 1% to 10% by weight trimethylene carbonate, from 12% to 20% by weight caprolactone, and from 1% to 10% by weight L-lactide.

9. The biocompatible adhesive of claim 8, wherein the lactone includes a random copolymer possessing 69% glycolide, 7% trimethylene carbonate, 17% caprolactone, and 7% lactide.

10. The biocompatible adhesive of any preceding claim, wherein the ratio of the ether to the lactone is about 2:1.

11. The biocompatible adhesive of any preceding claim, wherein the biocompatible adhesive further includes at least one bioactive agent.

12. A hernia patch comprising:
a mesh substrate having a surface; and
a coating on at least a portion of the surface of the mesh substrate, the coating including a biocompatible adhesive according to any preceding claim.

## Patentansprüche

1. Biokompatibler Klebstoff, umfassend:
eine erste Komponente, die mindestens ein lineares Polyethylenglykol, das ein Molekulargewicht von 1.000 bis 10.000 Dalton aufweist, in Kombination mit mindestens einem mehrarmigen Polyethylenglykol beinhaltet, das ein Molekulargewicht von 1.000 bis 3.000 Dalton aufweist; und
eine zweite Komponente, die ein Lacton beinhaltet.

2. Biokompatibler Klebstoff nach Anspruch 1, wobei die erste Komponente das mindestens eine lineare Polyethylenglykol und das mindestens eine mehrarmige Polyethylenglykol in einem Gewichtsverhältnis von 99:1 bis 80:20 beinhaltet.

3. Biokompatibler Klebstoff nach Anspruch 2, wobei die erste Komponente das mindestens eine lineare Polyethylenglykol und das mindestens eine mehrarmige Polyethylenglykol in einem Gewichtsverhältnis von 98:2 bis 88:12 beinhaltet.

4. Biokompatibler Klebstoff nach einem der vorstehenden Ansprüche, wobei das mehrarmige Polyethylenglykol vier bis zwölf Arme aufweist.

5. Biokompatibler Klebstoff nach Anspruch 4, wobei das mehrarmige Polyethylenglykol vier Arme aufweist.

6. Biokompatibler Klebstoff nach Anspruch 5, wobei das mindestens eine lineare Polyethylenglykol ein Molekulargewicht von etwa 4.600 Dalton aufweist und das mindestens eine mehrarmige Polyethylenglykol ein Molekulargewicht von etwa 2.000 Dalton aufweist.

7. Biokompatibler Klebstoff nach einem beliebigen vorhergehenden Anspruch, wobei das Lacton ausgewählt ist aus der Gruppe bestehend aus Lactid, Glycolid, Caprolactonen, Valerolactonen, Dioxanonen, Dioxepanonen, Trimethylencarbonaten, Propiolactonen, Butyrolacton und Kombinationen davon.

8. Biokompatibler Klebstoff nach einem beliebigen vorhergehenden Anspruch, wobei das Lacton ein quartäres Polymer ist, das 62 Gew.-% bis 72 Gew.-% Glykolid, 1 Gew.-% bis 10 Gew.-% Trimethylencarbonat, 12 Gew.-% bis 20 Gew.-% Caprolacton und 1 Gew.-% bis 10 Gew.-% L-Lactid beinhaltet.

9. Biokompatibler Klebstoff nach Anspruch 8, wobei das Lacton ein Random-Copolymer mit 69% Glykolid, 7% Trimethylenkarbonat, 17% Caprolacton und 7% Lactid beinhaltet.

10. Biokompatibler Klebstoff nach einem beliebigen vorhergehenden Anspruch, wobei das Verhältnis von Ether zu Lacton etwa 2:1 beträgt.

11. Biokompatibler Klebstoff nach einem beliebigen vorhergehenden Anspruch, wobei der biokompatible Klebstoff weiterhin mindestens einen bioaktiven Wirkstoff beinhaltet.

12. Hernienpflaster, umfassend:
ein Netzsubstrat, das eine Oberfläche aufweist; und
eine Beschichtung auf mindestens einem Abschnitt der Oberfläche des Netzsubstrats, wobei die Beschichtung einen biokompatiblen Klebstoff nach einem der vorstehenden Ansprüche beinhaltet.

## Revendications

1. Adhésif biocompatible comprenant :
un premier constituant incluant au moins un polyéthylène glycol linéaire ayant un poids moléculaire de 1000 à 10 000 Daltons, en combinaison avec au moins un polyéthylène glycol à bras multiples ayant un poids moléculaire de 1000 à 3000 Daltons ; et
un second constituant incluant une lactone.

2. Adhésif biocompatible selon la revendication 1, dans lequel le premier constituant inclut l'au moins un polyéthylène glycol linéaire et l'au moins un polyéthylène glycol à bras multiples en un rapport de 99:1 à 80:20 en poids.

3. Adhésif biocompatible selon la revendication 2, dans lequel le premier constituant inclut l'au moins un polyéthylène glycol linéaire et l'au moins un polyéthylène glycol à bras multiples en un rapport de 98:2 à 88:12 en poids.

4. Adhésif biocompatible selon l'une quelconque des revendications précédentes, dans lequel le polyéthylène glycol à bras multiples a quatre à douze bras.

5. Adhésif biocompatible selon la revendication 4, dans lequel le polyéthylène glycol à bras multiples a quatre bras.

6. Adhésif biocompatible selon la revendication 5, dans lequel l'au moins un polyéthylène glycol linéaire a un poids moléculaire d'environ 4600 Daltons et l'au moins un polyéthylène glycol à bras multiples a un poids moléculaire d'environ 2000 Daltons.

7. Adhésif biocompatible selon l'une quelconque des revendications précédentes, dans lequel la lactone est sélectionnée dans le groupe constitué par le lactide, le glycolide les caprolactones, les valérolactones, les dioxanones, les dioxépanones, les carbonates de triméthylène, les propiolactones, la butyrolactone, et les combinaisons de ceux-ci.

8. Adhésif biocompatible selon l'une quelconque des revendications précédentes, dans lequel la lactone est un polymère quaternaire incluant de 62 % à 72 % en poids de glycolide, de 1 % en poids à 10 % en poids de carbonate de triméthylène, de 12 % à 20 % en poids de caprolactone, et de 1 % à 10 % en poids de L-lactide.

9. Adhésif biocompatible selon la revendication 8, dans lequel la lactone inclut un copolymère statistique possédant 69% de glycolide, 7 % de carbonate de triméthylène, 17 % de caprolactone, et 7 % de lactide.

10. Adhésif biocompatible selon l'une quelconque des revendications précédentes, dans lequel le rapport de l'éther sur la lactone est d'environ 2:1.

11. Adhésif biocompatible selon l'une quelconque des revendications précédentes, dans lequel l'adhésif biocompatible comprend en outre au moins un agent bioactif.

12. Timbre pour hernie comprenant :
un substrat à mailles ayant une surface ; et
un revêtement sur au moins une partie de la surface du substrat à maille, le revêtement incluant un adhésif biocompatible selon l'une quelconque des revendications précédentes.
